# EUROPEAN PATENT APPLICATION

(11) **EP 4 349 992 A1**
(43) Date of publication of application: **10.04.2024**
(21) Application number: 22811598.6
(22) Date of filing: 24.05.2022
(51) Int. Cl.: C12N 15/85, C12N 9/64, G01N 33/558

(54) **CELLS SENSITIVE TO BOTULINUM TOXIN INTO WHICH SPECIFIC GENE HAS BEEN INSERTED BY LENTIVIRUS**

(30) Priority: 24.05.2021 KR 20210066040; 23.05.2022 KR 20220063008
(71) Applicant: ATGC Co., Ltd., Gangnam-gu Seoul 06372 (KR)
(72) Inventor: JANG, Sung Su, Seoul 05510 (KR); LIM, Il Ho, Seoul 05837 (KR); LEE, Hak Sup, Seongnam-si, Gyeonggi-do 13533 (KR); AHN, Yong Shik, Seoul 02582 (KR); CHOI, Doo Jin, Bucheon-si, Gyeonggi-do 14533 (KR)
(74) Representative: Nederlandsch Octrooibureau
(86) International application number: PCT/KR2022/007323
(87) International publication number: WO 2022/250405

(57) **Abstract**

The present invention relates to cells for measuring botulinum toxin activity overexpressing SEPTIN2 or Thioredoxin (TXN), and to a method for measuring botulinum toxin activity, the method comprising the steps of: treating the cells for measuring botulinum toxin activity with botulinum toxin and culturing the cells; lysing the cultured cells and collecting a cell lysate; and measuring the amount of SNAP-25 cleavage products in the cell lysate.

## Description

### [Technical Field]

The present invention relates to cells for measuring botulinum toxin activity, a method for measuring botulinum toxin activity using the same, and the like.

### [Background Art]

Botulinum toxin is a neurotoxin produced by *Clostridium botulinum*, which is a Gram-positive anaerobic bacterium that grows in rotting canned food and rotten meat. Botulinum toxin is classified into eight neurotoxins, and among them, seven (A, B, C, D, E, F, and G) may cause nerve paralysis. Botulinum toxin has a size of about 150 kDa and consists of a complex of non-toxin proteins in addition to the botulinum toxin protein, and the size of each complex can reach a maximum of 900 kDa depending on the type of neurotoxin. The mode of action, target, period of activity, and the like vary depending on the type of botulinum toxin, but botulinum toxin type A is known to be one of the most lethal biological agents. This botulinum toxin has an action of causing paralysis by blocking signals that induce muscle spasms or contractions, and was approved by the US FDA in 1989 as a biological agent based on this function, and since then, the botulinum toxin has been widely used for therapeutic and cosmetic purposes. Botulinum toxin is used for therapeutic purposes to treat diseases such as strabismus, torticollis, blepharospasm, achalasia, anal fissures, and back pain, and for cosmetic purposes to remove wrinkles and frown lines, reduce square jaws, and treat hyperhidrosis, and the like.

Therapeutic and cosmetic uses of botulinum toxin require biological validation before use, and such validation is generally confirmed through a mouse LD₅₀ using mice, that is, a lethality test. In practice, the units on the label of a pharmaceutical preparation are mouse LD₅₀ units. However, in order to provide statistically useful mouse LD₅₀ data, there are limitations, such as not only the very large number of mice required, but also the high cost for testing, and difficulty in observing differences depending on the serotype of botulinum toxin (Korean Patent Publication No. 10-2012-0134154).

Therefore, in order to overcome these disadvantages, there is a need for a new method for measuring botulinum toxin activity, which is simple and highly sensitive, and can evaluate all the steps required for the absorption of botulinum toxin without using animals.

### [Disclosure]

### [Technical Problem]

The present invention has been devised to solve the problems in the related art as described above, and an object thereof is to provide cells for measuring botulinum toxin activity, a method for measuring botulinum toxin activity using the same, and the like. The cells are cells that overexpress SEPTIN2 or thioredoxin (TXN) genes, and may verify even low doses of botulinum toxin with high accuracy because not only binding, cellular uptake, translocation into the cytoplasm, and protease activity of botulinum toxin can all be evaluated, but also the sensitivity to botulinum toxin has been remarkably improved. The activity of botulinum toxin pharmaceutical compositions in Active Pharmaceutical Ingredient and finished drug states (Finished Dosage Form) may also be measured.

However, the technical problems which the present invention intends to solve are not limited to the technical problems, which have been mentioned above, and other technical problems, which have not been mentioned, will clearly be understood by those with ordinary skill in the art to which the present invention pertains from the following description.

### [Technical Solution]

The present invention provides cells for measuring botulinum toxin activity, overexpressing SEPTIN2 or thioredoxin (TXN).

In an exemplary embodiment of the present invention, a SEPTIN2 or TXN gene may be overexpressed by inserting the gene into the cells, preferably by a method such as transduction or transfection, but the present invention is not limited to the method as long as the method is a generally known method for inserting genes into cells. The SEPTIN2 is preferably a human SEPTIN2 gene, more preferably a gene expressing the 3,726 bp mRNA sequence of accession number NM_001008491.2 of NCBI, but variants thereof are also included in the scope of the present invention. Specifically, the SEPTIN2 may include a sequence having a sequence homology of 90% or more, more preferably 95% or more, and most preferably 98 % or more with the mRNA sequence of NM_001008491.2. The % sequence homology is confirmed by comparing a comparison region with an optimally aligned sequence, and a portion of the nucleotide sequence in the comparison region may include an addition or deletion (that is, a gap) compared to a reference sequence (without an addition or deletion) for the optimal alignment of the sequences. Moreover, the TXN is preferably a human TXN gene, more preferably a gene expressing the 737 bp mRNA sequence of accession number NM_003329.4 of NCBI, but variants thereof are also included in the scope of the present invention. Specifically, the TXN may include a sequence having a sequence homology of 90% or more, more preferably 95% or more, and most preferably 98 % or more with the mRNA sequence of NM_003329.4. The % sequence homology is confirmed by comparing a comparison region with an optimally aligned sequence, and a portion of the nucleotide sequence in the comparison region may include an addition or deletion (that is, a gap) compared to a reference sequence (without an addition or deletion) for the optimal alignment of the sequences.

In another exemplary embodiment of the present invention, the cells are characterized by having increased sensitivity to botulinum toxin intoxication compared to wild type cells due to the overexpression of the SEPTIN2 or TXN gene.

In still another exemplary embodiment of the invention, the cells may be preferably SiMa cells, LAN-2 cells, PC12 cells, Neuro-2a cells, LA1-55n cells, N18 cells, SH-SY5Y cells, Kelly cells, NB69 cells, N1E-115 cells, BE(2)-M17 cells, SK-N-BE(2) cells, and the like, but are not limited thereto as long as they are a cell line that is generally known to be usable for measuring botulinum toxin activity.

In yet another exemplary embodiment of the present invention, the botulinum toxin may be selected from the group consisting of botulinum serotypes A, B, C, D, E, F, and G.

Further, the present invention provides a method for measuring botulinum toxin activity, the method including the steps of: a) treating the cells for measuring botulinum toxin activity with botulinum toxin and culturing the cells; b) lysing the cultured cells and collecting a cell lysate; and c) measuring the amount of SNAP-25 cleavage product in the cell lysate.

In an exemplary embodiment of the present invention, the amount of SNAP-25 cleavage product is measured using a sandwich immunoassay (ELISA), a Western blot, and the like, but the method is not limited thereto, as long as it is a known method used to detect proteins.

In addition, the present invention provides a kit for measuring botulinum toxin activity, including the cells for measuring botulinum toxin activity as an active ingredient.

Furthermore, the present invention provides a use of the cells for measuring botulinum toxin activity for measuring botulinum toxin activity.

### [Advantageous Effects]

Since the cells for measuring botulinum toxin activity according to the present invention have remarkably improved sensitivity to botulinum toxin by overexpressing SEPTIN2 or thioredoxin, the cells can measure even low doses of botulinum toxin can be verified with high accuracy. In addition, it is expected that the cells can replace numerous animal experiments because binding, cellular uptake, translocation into the cytoplasm, and protease activity of botulinum toxin can all be evaluated. Further, since the cells can be used to measure not only botulinum toxin activity, but also the activity of botulinum toxin pharmaceutical compositions in Active Pharmaceutical Ingredient and Finished Dosage Form, it is expected that the cells can be easily applied to various fields of industries using botulinum toxin.

### [Description of Drawings]

FIG. 1 is a view schematically illustrating the functions of SEPTIN2 and thioredoxin (TXN).
FIG. 2 is a view schematically illustrating a pLenti-C-Myc-DDK-P2A-Puro vector map.
FIG. 3 is a view illustrating the results of confirming the cultured 293FT cell line according to an exemplary embodiment of the present invention under a microscope.
FIG. 4 is briefly a view illustrating the method for producing a cell line transduced using a lentivirus according to an exemplary embodiment of the present invention.
FIG. 5 is a view illustrating a method of separating and culturing a cell line into individual colonies using a cloning cylinder according to an exemplary embodiment of the present invention.
FIG. 6 is a view illustrating the results of confirming proteins expressed in a transduced cell line according to an exemplary embodiment of the present invention by Western blotting.
FIG. 7 is a view illustrating binding positions of primers prepared for PCR according to an exemplary embodiment of the present invention.
FIG. 8 is a view illustrating the results of confirming the introduced gene according to an exemplary embodiment of the present invention by PCR.
FIG. 9 is a view schematically illustrating the principle of sandwich ELISA.
FIG. 10 is a view illustrating the results of confirming the sensitivity to botulinum toxin between a control and a cell line transduced with a SEPTIN2 gene according to an exemplary embodiment of the present invention through the comparison of the amount of SNAP-25 cleavage.
FIG. 11 is a view illustrating the results of confirming the sensitivity to botulinum toxin between a control and a transduced cell line according to an exemplary embodiment of the present invention through the raw data ratio between each group.
FIG. 12 is a view illustrating the results of confirming the sensitivity to botulinum toxin between a control and a cell line transduced with a thioredoxin gene according to an exemplary embodiment of the present invention through the comparison of the amount of SNAP-25 cleavage.
FIG. 13 is a view illustrating the results of measuring the activity of a botulinum toxin pharmaceutical composition (Active Pharmaceutical Ingredient), using a transduced cell line, as the titer of the botulinum toxin pharmaceutical composition (Active Pharmaceutical Ingredient) according to an exemplary embodiment of the present invention.
FIG. 14 is a view illustrating the results of measuring the activity of a botulinum toxin pharmaceutical composition (Finished Dosage Form), using a transduced cell line, as the titer of the botulinum toxin pharmaceutical composition (Finished Dosage Form) according to an exemplary embodiment of the present invention.
FIG. 15 is a view schematically illustrating the overall development process of the present invention.

### [Modes of the Invention]

As a result of intensive studies on methods for measuring botulinum toxin activity, the present inventors confirmed that a cell line with improved sensitivity capable of measuring botulinum toxin activity was produced, and botulinum toxin activity could be stably measured using the same, thereby completing the present invention.

As used herein, the "botulinum toxin" refers to a type of botulinum toxin that may be produced by bacteria or produced by a recombinant technique, but includes any known type of botulinum toxin or any type of botulinum toxin subsequently discovered, including modified variants or fusion proteins. The botulinum toxin is classified into eight neurotoxins, and seven of botulinum toxin serotypes A, B, C, D, E, F, and G may cause nerve paralysis. This protein is classified into a protein that includes a complex and a protein that does not include a complex, and a pure toxin protein has a molecular weight of 150 KDa, and various proteins with 300 KDa, 500 KDa, and 900 KDa are produced according to whether the complex is formed. The botulinum toxin of the present invention may alternatively be a botulinum toxin derivative, that is, a compound that has botulinum toxin activity, but includes one or more chemical modifications or functional modifications as compared to a natural or recombinant botulinum toxin. For example, the botulinum toxin may be a modified neurotoxin (for example, a neurotoxin having one or more amino acid deletions, modifications, or substitutions as compared to a wild type neurotoxin or neurotoxin produced by recombination, derivatives thereof, or fragments thereof). For example, the botulinum toxin may be modified in a way that enhances characteristics thereof or reduces undesirable side effects thereof, but still retains desired botulinum toxin activity. Alternatively, the botulinum toxin may be a toxin produced using a recombinant or synthetic chemical technique (for example, a recombinant peptide, a fusion protein, or a hybrid neurotoxin prepared from different botulinum toxin serotype subunits or domains (see, for example, US Patent No. 6,444,209)). The botulinum toxin may also be a part of the overall molecule that has been proven to have the required botulinum toxin activity, and in such cases, it may be used on its own or as a part of a combination or conjugate molecule, for example, a fusion protein. Further, the botulinum toxin may be in the form of a precursor for botulinum toxin, which may itself be non-toxic, for example, a non-toxic zinc protease that may become toxic upon proteolytic degradation.

As used herein, "cells" refer to all eukaryotic cells that are susceptible to botulinum toxin intoxication by botulinum toxin or that are capable of absorbing botulinum toxin. Eukaryotic cells refer to cells derived from various mammals such as, for example, mice, rats, pigs, cows, sheep, horses, primates, and humans. As used herein, a prepared cell line is synonymous with an established cell line, an immortal cell line, or a transformed cell line, and refers to cells selected for unlimited proliferation. The transformed cell lines disclosed in the present specification exhibit consistent sensitivity to botulinum toxin activity over a plurality of cell passages, and sensitivity to botulinum toxin activity refers to the lowest botulinum toxin concentration at which a signal detected by the untreated control group or background signal can be consistently measured.

As used herein, the "vector" or "plasmid" refers to a DNA fragment, nucleic acid molecule, and the like, which are delivered into a cell, and the vector may replicate DNA and be independently re-manufactured in a host cell. The vector may be used interchangeably with the term "carrier." "Expression vector" refers to a recombinant DNA molecule that includes a target coding sequence and an appropriate nucleic acid sequence that is essential for expressing an operably linked coding sequence in a specific host organism. In addition, the recombinant vector or plasmid of the present invention is a general term for all vectors that include a gene encoding SEPTIN2 or thioredoxin, and are capable of overexpressing SEPTIN2 or thioredoxin in cells.

As used herein, a kit refers to a device capable of measuring the activity of botulinum toxin by including the cells for measuring botulinum toxin activity of the present invention, and may be used to measure the activity of botulinum toxin such as botulinum toxin itself, an Active Pharmaceutical Ingredient of botulinum toxin, and a Finished Dosage Form of botulinum toxin, and the kit of the present invention may further include a cell lysing agent for lysing cells, antibodies and reagents for ELISA, a manual, and the like, in addition to the cells for measuring botulinum toxin activity of the present invention, but may further include anything as long as it can be used for the method for measuring botulinum toxin activity of the present invention.

Hereinafter, preferred examples for helping the understanding of the present invention will be suggested. However, the following examples are provided only so that the present invention may be more easily understood, and the content of the present invention is not limited by the following examples.

### [Examples]

### Example 1: Culture of cell line 293FT for lentivirus production

293FT, a cell line commonly used for lentivirus production, is derived from a 293F cell line, and is a typical cell line used for lentivirus production as a cell line stably expressing an SV40 large T antigen through a pCMVSPORT6TAg.neo plasmid. Since the 293FT cell line encodes a neomycin resistance gene in the plasmid, the 293 cell line was cultured using a medium including geneticin, which is a neomycin analog, during all of the cell culture except for cell thawing. More specifically, a 293FT cell stock (Thermo, R700-07) was suspended in a DMEM (Gibco) medium supplemented with 10% FBS (Gibco), 1X NEAA (Gibco), 2 mM L-glutamine (Gibco) and 1% penicillin-streptomycin (Gibco), and centrifuged at 200xg for 3 minutes to remove the supernatant and obtain cells. The obtained cells were re-suspended using a culture medium, and then dispensed into a 100-mm cell culture dish and cultured at 37°C in a 5% CO₂ incubator for 24 hours. Thereafter, the culture medium was completely removed, and replaced with a DMEM (Gibco) medium supplemented with 10% FBS (Gibco), 1X NEAA (Gibco), 2 mM L-glutamine (Gibco), 1% penicillin-streptomycin (Gibco) and 500 µg/mL of the geneticin^{®} selective antibiotic (Gibco), and then the cells were cultured. Then, when cell confluency increased to 90% or more, sub-culture was performed again. For the sub-culture, the existing culture medium was first removed, and the cell culture dish was washed by adding DPBS (Gibco) in an amount of 5 mL, which is 50% of the culture volume. Then, the DPBS was removed, TrypLE (Gibco) was added in an amount of 2 mL, which is 20% of the culture volume, and the mixture was reacted at 37°C in a 5% CO₂ incubator for 2 minutes. After the reaction was completed, additional culture medium was added, and then the supernatant was removed by centrifugation at 800xg and 4°C for 2 minutes. Then, after the cells were re-suspended using the culture medium, the number of cells was measured using trypan blue (Gibco) and a hematocytometer, 2×10⁶ cells were dispensed into a 100 mm cell culture dish with 10 mL of a culture medium, and sub-culture was performed every 3 to 4 days using the method described above.

### Example 2: Selection of gene and production of encoding plasmid to produce cell lines vulnerable to BoNT/A intoxication

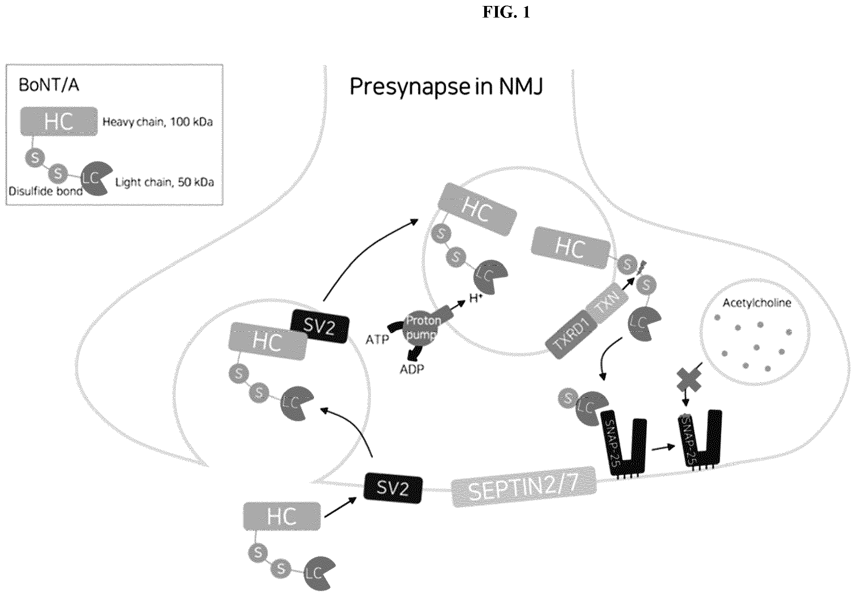

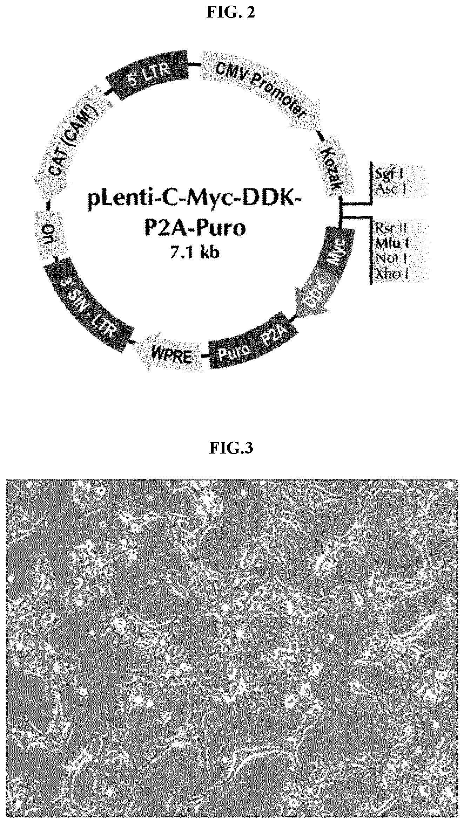

As shown in FIG. 1, it is known that SEPTIN2 (SEPT2) serves to protect BoNT/A from being degraded in cells and thioredoxin (TXN) acts to separate a disulfide bond between the heavy and light chains of BONT/A. In order to overexpress the two proteins in cells, a pLenti-ORF plasmid (SEPTIN2: RC224864L3, TXN: RC208876L3) in which genes encoding each protein were inserted into a pLenti-C-Myc-DDK-P2A-Puro vector was produced by commissioning OriGene Technologies, Inc. For the production of SEPTIN2, an amino acid sequence of SEQ ID NO: 9 (DNA sequence: SEQ ID NO: 10) was used, and for the production of thioredoxin, an amino acid sequence of SEQ ID NO: 11 (DNA sequence: SEQ ID NO: 12) was used. The pLenti-C-Myc-DDK-P2A-Puro vector map is shown in FIG. 2. After a tube containing the lyophilized pLenti-ORF plasmid was centrifuged at 5000xg for 3 minutes, 100 µL of distilled water was added thereto, and the resulting mixture was pipetted, and then stored in a freezer at - 20°C until use.

To produce a bacterial strain transformed with the pLenti-ORF plasmid, the plasmid was thawed at room temperature, and 100 µL of competent cells (RBC Bioscience) were thawed at 4°C. Then, the competent cells were treated with 2 µL of the thawed plasmid, reacted at 4°C for 10 minutes, and then heat shock was applied thereto at 37°C for 1 minute, 700 µL of LB broth was added, and the cells were cultured in a shaking incubator at 37°C for 15 minutes. After the culture was completed, 20 µL of the culture solution was added to an LB agar plate supplemented with 34 µg/mL chloramphenicol and spread evenly with a spreader. The agar plate treated with bacteria was incubated in an incubator at 37°C for 16 hours. Then, each colony generated on the agar plate was inoculated into 1.5 mL of LB broth supplemented with chloramphenicol, and shake-cultured in an incubator at 37°C for 16 hours. After the culture was completed, the culture solution was centrifuged at 13,000 rpm for 1 min to remove the supernatant, and the remaining pellet was used for the purification of the plasmid according to the provided protocol using a DNA-spin plasmid DNA purification kit (iNtRON). Then, the base sequence of the purified plasmid was analyzed by commissioning Cosmogenetech Co., Ltd. Primer sequences used for nucleotide sequencing are shown in Table 1. As a result of nucleotide sequencing, it was confirmed that SEPTIN2 exactly matches the accession number NM_001008491.2 of NCBI, and TXN exactly matches NM_003329.4, and through this, it was confirmed that a plasmid into which SEPTIN2 or TXN, that is, a target gene, had been normally inserted was constructed.

**[Table 1]**

| Name | Primer sequence (5' -> 3') | SEQ ID NO: |
|---|---|---|
| SEPTIN2 V2 | AGAGCTCGTTTAGTGAA | 1 |
| SEPTIN2-600F | GGATGAAATTGAAGAACATA | 2 |
| TXN V2 | AGAGCTCGTTTAGTGAA | 3 |

To mass-produce the pLenti-ORF plasmid, 500 µL of the culture solution of the strain transformed with the plasmid was inoculated into 100 mL of LB broth supplemented with 34 µg/mL chloramphenicol, and then shake-cultured at 37°C for 16 hours. Then, the culture solution was transferred to a 500-mL centrifuge bottle (Nalgene) and centrifuged at 6000xg and 4°C for 15 minutes, and the supernatant was removed. Then, the plasmid was purified from the pellet using a HiSpeed Plasmid Midi Kit (Qiagen). The purified plasmid was quantified using Life Science UV/Vis Spectrophotometer DU730 (Beckman Coulter). The results are shown in Table 2.

**[Table 2]**

| Gene | A260 | A280 | 260/280 ratio | DNA concentration (ng/µL) |
|---|---|---|---|---|
| TXN | 1.119 | 0.595 | 1.880 | 559 |
| SEPTIN2 | 1.059 | 0.562 | 1.884 | 529 |

### Example 3: Production of lentivirus by transfection in 293FT cell line

2.5 × 10⁶ cells of 293FT cell line, which is a cell line with high lentivirus production efficiency, sub-cultured in the same manner as in Example 1 were dispensed into a 100-mm cell culture dish. Then, it was confirmed under a microscope that the cell confluency reached 40 to 50%. The results are shown in FIG. 3. Then, for transfection, 1.5 mL of opti-MEM (Gibco) and 5 µg of pLenti-ORF plasmid were added to a 1.5-mL tube and mixed. Then, 6 µg of 0.5 µg/µL packaging plasmid (OriGene) contained in distilled water was added and mixed, and then 33 µL of TurboFectin (OriGene) was additionally added and mixed. After the mixed solution was allowed to react at room temperature for 15 minutes, the cell culture dish was completely treated with the mixed solution and incubated for 2 days. Then, a medium supernatant including the virus was collected and stored at 4°C, and a fresh medium was added thereto, and then the cells were again cultured for 1 day. Then, the medium supernatant was again collected and mixed with the medium collected the previous day, and the resulting mixture was filtered using a 0.45-µm pore filter (syringe filter, Sartorius AG), and stored at 4°C until use.

### Example 4: Production of transduced cell line using lentivirus

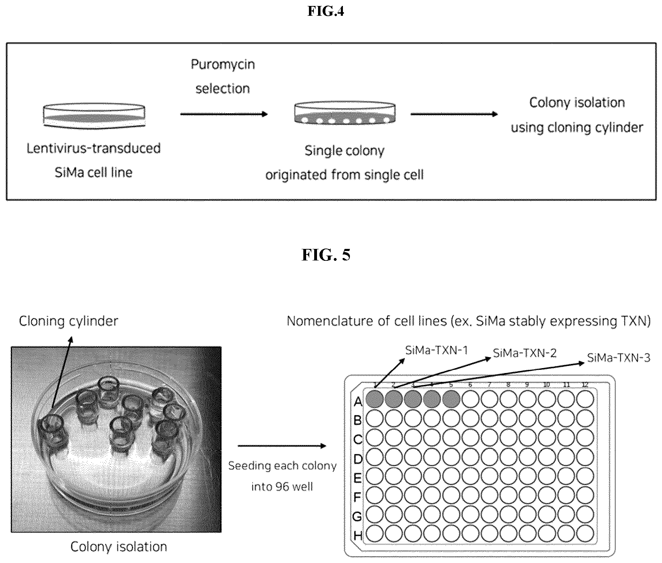

4×10⁶ SiMa (DSMZ, ACC164) cell lines sub-cultured in the same manner as in Example 1 were dispensed into a 60-mm cell culture dish to which 4 mL of a RPMI1640 (Gibco) culture medium supplemented with 10% FBS, 2 mM L-glutamine (Gibco), and 1% penicillin-streptomycin (Gibco) was added, and cultured for 16 hours. Then, after the culture solution was removed, 1 mL of the lentivirus solution obtained in the same manner as in Example 3 and 3 mL of a fresh culture medium were mixed and added, polybrene (Sigma-Aldrich) was added to make a final concentration of 8 µg/mL, and the cells were cultured. The next day, the culture solution was removed, a fresh medium was added, and then again incubated for 1 day and replaced with a fresh culture medium supplemented with 1 µg/mL puromycin (Sigma-Aldrich). Thereafter, only transduced puromycin drug-resistant cells were selected while replacing the culture medium with a puromycin-containing culture medium at 3 to 4 day intervals. The method for producing a cell line transduced using a lentivirus is schematically shown in FIG. 4.

Then, in order to separate the cultured cell lines into individual colonies, a cloning cylinder (Sigma-Aldrich) was used to physically divide the space in the culture dish, as shown in FIG. 5. More specifically, the medium used for culture was removed, the cells were treated with 3 mL of DPBS and washed, a cloning cylinder was attached to each colony, and only the DPBS in the cylinder was separately removed. Then, the cloning cylinder was each treated with 50 µL of trypLE and was allowed to react in a 5% CO₂ incubator at 37°C for 3 minutes. Then, trypLE and a cell mixture solution were mixed by pipetting and then dispensed into a 96-well plate containing 150 µL of a culture medium supplemented with 1 µg/mL puromycin. Then, the cell line was named in the dispensed order. Thereafter, the cells were scaled up and cultured according to the growth of the cells.

### Example 5: Confirmation of transduced cell lines

### 5.1. Verification of expression of introduced protein

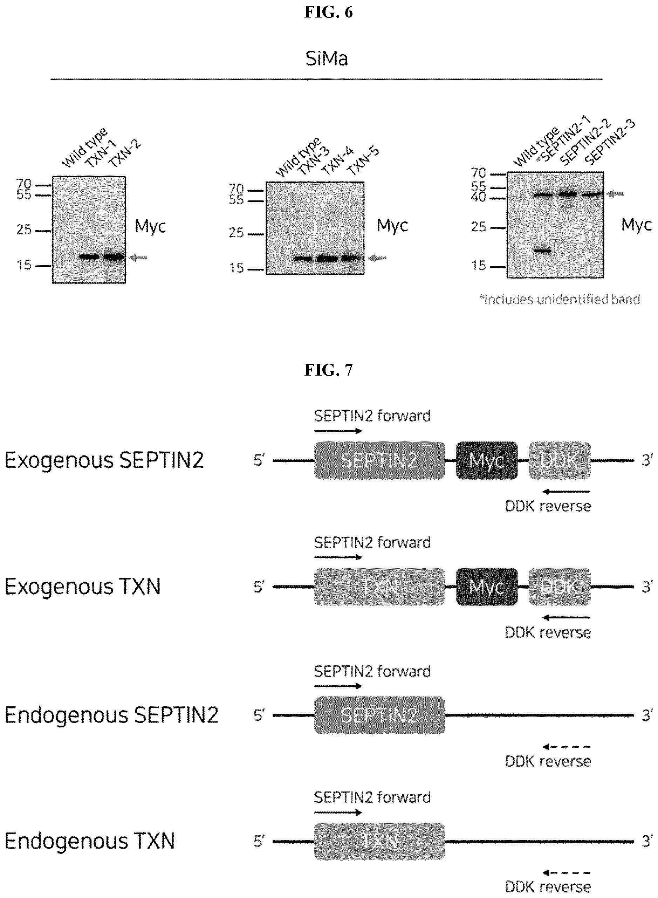

In order to confirm a transduced cell line produced in the same manner as in Example 4, a primarily expressed protein was confirmed using Western blotting. More specifically, the medium of the 100 mm cell culture dish that had been scaled up and cultured was removed, and the cells were washed using 5 mL of DPBS at 4°C. Then, after the DPBS was removed, cells were treated with 200 µL of RIPA lysis buffer (iNtRON) supplemented with cOmplete^{™} and EDTA-free Protease Inhibitor Cocktail (Roche). A cell lysate was transferred to a 1.5 mL tube using a cell lifter (SPL), and reacted without movement at 4°C for 20 minutes. Then, after centrifugation under the conditions of 17,000 rpm and 4°C for 30 minutes, the supernatant was transferred to a new 1.5 mL tube. The amount of proteins in the supernatant was quantified using a Pierce^{™} BCA Protein Assay Kit (Thermo Fisher), sampling was performed with 4X Laemmli Sample Buffer (Bio-Rad), and then heating was performed at 100°C for 10 minutes to prepare a sample for Western blotting. Then, western blotting was performed using a 15% polyacrylamide gel. Proteins in the sample were separated by size through electrophoresis and transferred to an immobilon-PPVDF membrane (Merck) at 100 V for 1 hour. A protein expression pattern was confirmed by staining the membrane onto which proteins were completely transferred with Ponceau S (Sigma-Aldrich). Then membrane was immersed in 0.1% polysorbate 20 in PBS (PBST) and washed 4 times using a digital orbital shaker (DAIHAN Scientific) for 5 minutes each to remove the Ponceau S, and a blocking buffer (5% BSAPBST) was added thereto, and blocking was performed at room temperature for 1 hour using the digital orbital shaker. Both Myc and DDK tags are expressed at the C-terminus of an expressed protein when a target gene in the PLenti-C-Myc-DDK-P2A-Puro vector, that is, an inserted gene, is expressed. Therefore, the membrane for which blocking had been completed was treated with a primary antibody that specifically binds to the Myc tag (Myc-tag, Cell Signaling Technology, 2278S, 1:1000 v/v in 2% BSA PBST), and reacted at 4°C for 16 hours using a digital orbital shaker. The membrane on which reaction had been completed was immersed in PBST, washed 4 times for 5 minutes each using a digital orbital shaker, then treated with a secondary antibody (Anti-Rabbit HRP, abcam, ab6721, 1: 10000 v/v in PBST), and reacted at room temperature for 1 hour. Thereafter, the membrane was immersed in PBST and washed 4 times for 5 minutes each using a digital orbital shaker, and treated with a Pierce ECL Western Blotting Substrate (Thermo Fisher), and proteins were detected with ImageQuant LAS 500 (Cytiva). The results are shown in FIG. 6.

As shown in FIG. 6, it was confirmed that the thioredoxin protein or Septin-2 protein was expressed in the transduced SiMa cell line. However, in the SEPTIN2-1 cell line, other unidentified bands in addition to Septin-2 were detected.

### 5.2. Verification of gene introduction

In order to confirm the introduction of the target gene in a transduced cell line produced in the same manner as in Example 4, genomic DNA was isolated from the cell line, and then the introduced gene portion was amplified using PCR. A primer for PCR was designed to distinguish between a transduced gene and an endogenous gene using the sequence of the 3'-terminus, which has only transduced genes, and then produced by commissioning Cosmogenetech Co., Ltd. The primer sequences used for PCR are shown in Table 3. As shown in FIG. 7, the forward primer for amplifying the TXN or SEPTIN2 gene was designed to bind to the 5'-terminal sequence of each gene, and the reverse primer common to the two genes was designed to bind to the DDK-tag sequence. Moreover, primers (GH20 and GH21) capable of amplifying the beta-globin gene were used as positive controls.

**[Table 3]**

| Purpose | Name | Primer sequence (5' -> 3') | SEQ ID NO: |
|---|---|---|---|
| PCR | TXN forward | CTTTTCAGGAAGCCTTGG | 4 |
| | SEPTIN2 forward | GTCTAAGCAACAGCCAAC | 5 |
| | DDK reverse (common) | CTTATCGTCGTCATCCTTG | 6 |
| Positive control | GH20 | GAAGAGCCAAGGACAGGTA C | 7 |
| | GH21 | GGAAAATAGACCAATAGGCA G | 8 |

For PCR, when transduced SiMa-TXN-3, SiMa-SEPTIN2-1, SiMa-SEPTIN2-2, and SiMa-SEPTIN2-3 cell lines were each dispensed into a cell culture dish containing a culture medium supplemented with 1 µg/mL puromycin and cell confluency reached about 80%, genomic DNA of each cell line was extracted according to the provided protocol using a Wizard^{®} Genomic DNA Purification Kit (Promega), and the extracted genomic DNA was quantified using Life Science UV/Vis Spectrophotometer DU730 (Beckman Coulter). The results are shown in Table 4.

**[Table 4]**

| Cell line | 260/280 ratio | DNA concentration (ng/uL) |
|---|---|---|
| SiMa-TXN-3 | 1.937 | 1072.0 |
| SiMa-SEPTIN2-1 | 1.830 | 887.5 |
| SiMa-SEPTIN2-2 | 1.939 | 947.0 |
| SiMa-SEPTIN2-3 | 1.916 | 1053.0 |

Then, after a PCR sample was prepared by mixing 50 ng of genomic DNA, 25 µL of 2X Platinum SuperFi PCR Master Mix, and 2.5 µL of each of 10 µM forward primer and reverse primer and adding distilled water to make a final volume of 50 µL, PCR was performed. PCR conditions are shown in Table 5.

**[Table 5]**

| Cycle | Procedure | Temperature (°C) | Time |
|---|---|---|---|
| 1 | Denaturation | 95 | 30 sec |
| 30 | Denaturation | 95 | 30 sec |
| | Annealing | 50 | 30 sec |
| | Elongation | 72 | 1 min 30 sec |
| 1 | Elongation | 72 | 5 min |
| 1 | Storage | 4 | Overnight |

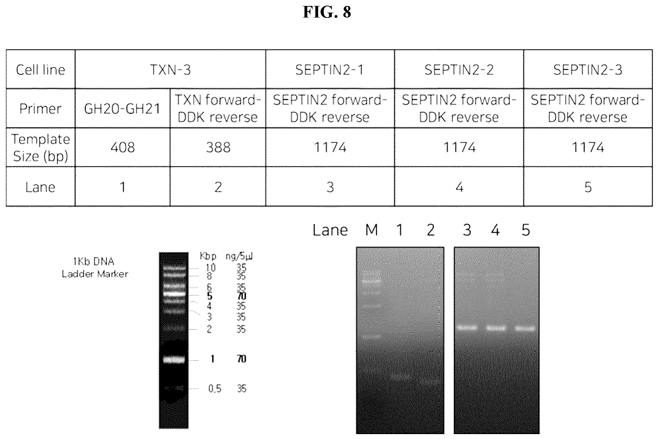

After the completion of PCR, the sample was electrophoresed using a 1.5% (w/v) DNA agarose gel mixed with a 0.01% (v/v) EcoDye^{™} Nucleic Acid Staining Solution (Biofact) and Mupid-One (Advance), which is an electrophoresis kit. Electrophoresis was performed at 100V for 35 minutes and imaged using Gel Documentation System LSG 1000 (iNtRON). The results are shown in FIG. 8.

As shown in FIG. 8, it was confirmed that a beta globin gene corresponding to the positive control and a TXN gene containing a transduced DDK-tag were both successfully amplified in the SiMa-TXN-3 cell line. Furthermore, it was confirmed that the SEPTIN2 genes including the transduced DDK-tag were all amplified even in each cell line of SiMa-SEPTIN2-1, SiMa-SEPTIN2-2, and SiMa-SEPTIN2-3. Through the results, it could be confirmed that a transduced cell line overexpressing the transduced gene was successfully produced.

### Example 6: Verification of BoNT/A biological activity by sandwich ELISA

### 6.1. Preparation of cell lysate of SiMa-SEPTIN2-3 cells treated with BoNT/A

In order to confirm a system for verifying the biological activity of a cell-based botulinum toxin, a cell lysate of cells treated with botulinum toxin was first prepared. To treat cells with botulinum toxin, a SiMa cell line as a control was dispensed into a 96-well plate containing a RPMI1640 medium supplemented with 10% FBS, 2 mM L-glutamine, and 1% penicillin-streptomycin at a concentration of 1.2 × 10⁵ cells/100 µL/well, and a SiMa-SEPTIN2-3 cell line as an experimental group was dispensed into a 96-well plate containing a RPMI1640 medium supplemented with 10% FBS, 2 mM L-glutamine, 1% penicillin-streptomycin, and 1 µg/mL puromycin at a concentration of 1.2 × 10⁵ cells/100 µL/well. After each cell line was dispensed and cultured for 2 days, the media were removed and the cell line was treated with 100 µL of a differentiation medium supplemented with 1X B-27^{™} Plus Supplement (Gibco), 1X N-2 Supplement (Gibco), 2 mM L-glutamine and 25 µg/mL trisialoganglioside GT_{1B} (Matreya) and cultured for 2 days to induce differentiation. After 2 days of differentiation, the medium was removed and a BoNT/A complex was serially diluted 1.35-fold in RPMI1640 supplemented with 1X B-27^{™} Plus Supplement, 1X N-2 Supplement, and 0.25% Human Serum Albumin (GC Corp.) and added to each well to make a final volume of 100 µL. After cells were treated with botulinum toxin and cultured for 4 days, the cells were lysed by removing the medium and treating each well with 110 µL of a lysis buffer supplemented with a protease inhibitor cocktail (50 mM HEPES (pH 7.4), 150 mM NaCl, 1.5 mM MgCl₂ and 1% Triton X-100). Then, the cell lysate was transferred to a 1.5 mL tube and centrifuged at 17,000 rpm and 4°C for 5 minutes to obtain the supernatant, thereby preparing a cell lysate for use in sandwich ELISA.

### 6.2. Confirmation of EC50 using sandwich ELISA

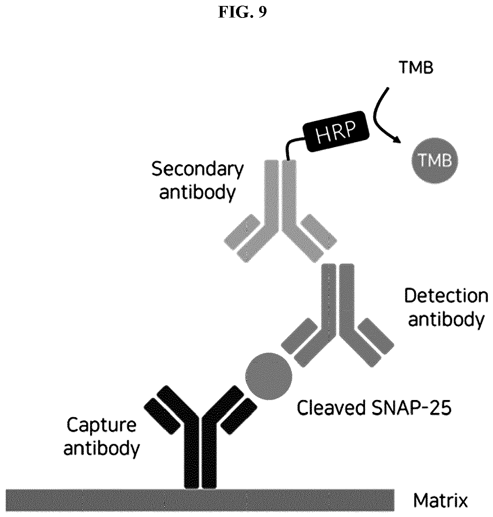

Since botulinum toxin serotype A (BoNT/A) is known to act on the presynapse of a neuromuscular junction to cause the cleavage of a 25 kDa synaptosomal-associated protein molecule (SNAP-25) bound to the presynaptic cell membrane, a sandwich enzyme-linked immunosorbent assay (ELISA) test method using cleaved SNAP-25 by applying this principle and each antibody capable of recognizing intact SNAP-25 was designed. The principle is schematically illustrated in FIG. 9.

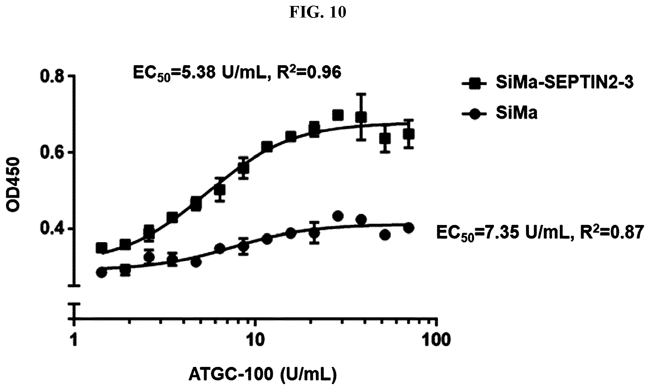

After a 25 kDa mouse synaptosomal protein (SNAP-25, a.a. 183-197) cleavage monoclonal antibody (Mybiosource, MBS312597), which is a capture antibody that specifically binds to cleaved SNAP-25, was diluted to a concentration of 0.2% (v/v) using a 0.1 M sodium carbonate coating buffer (pH 9.6), each well of a clear flat-bottom immuno nonsterile 96-well plate (Thermo Fisher) was treated with 100 µL of the antibody, and reacted without movement at 4°C for 16 hours. Thereafter, antibodies not bound to the wells were removed, and the wells were washed three times using 200 µL of 0.1% polysorbate 20 in PBS (PBST). Then, each well was treated with 200 µL of a blocking buffer (5% BSA PBST), and placed in a shaker under the conditions of room temperature and 75 rpm for 1 hour. After 1 hour, the blocking buffer was removed, the cells were washed three times using 200 µL of PBST, 90 µL of the cell lysate obtained in the same manner as in Example 6.1 was added, and the cells were reacted in a shaker under the conditions of room temperature and 75 rpm for 2 hours. Then, the cell lysate was removed, and the wells were washed three times with 200 µL of PBST. Thereafter, after an anti-SNAP-25 antibody (Sigma-Aldrich, S9684), which is a detection antibody, was diluted to a concentration of 0.1% (v/v) using a blocking buffer, each well was treated with 100 µL of the diluted antibody, and reacted in a shaker under the conditions of room temperature and 75 rpm for 1 hour. Thereafter, all unbound antibodies were removed by washing three times using 200 µL of PBST, anti-rabbit HRP (abcam, ab6721), which is a secondary antibody, was diluted to 0.01% (v/v) using a blocking buffer, and each well was treated with 100 µL of the diluted antibody solution and reacted in a shaker under the conditions of room temperature and 75 rpm for 1 hour. Then, after washing three times using 200 µL of PBST, the TMB solution of a TMB peroxidase EIA substrate kit (Bio-Rad) and a hydrogen peroxide solution were mixed at a ratio of 9:1, and each well was treated with 50 µL of the mixed solution, and then reacted in an incubator at 37°C for 30 minutes. Thereafter, after each well was treated with 50 µL of 2N sulfuric acid, the absorbance (OD450) at 450 nm was measured using a SpectraMax Plus 384 Microplate Reader (Molecular Devices). A dose-response curve (four parameters) was drawn from acquired raw data using GraphPad Prism Version 7.00 (GraphPad Software, Inc.), and EC₅₀ values were calculated based on in-house mouse potency test results. Thereafter, all experiments were repeated at least three times, and the results were expressed as mean ± standard deviation. The results are shown in FIG. 10.

As shown in FIG. 10, it was confirmed that the EC₅₀ of the SiMa cell line, which is a control, exhibited a value of 7.35 U/mL (0.49 pM) and the EC₅₀ of the SEPTIN2-3 transduced cell line exhibited a value of 5.38 U/mL (0.36 pM). Through this, it could be confirmed that sensitivity could be improved by overexpressing SEPTIN2.

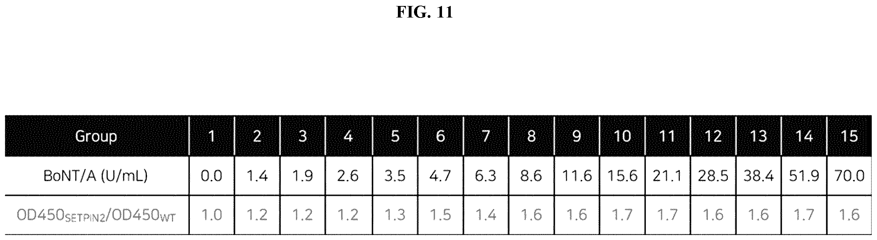

In addition, in order to compare the difference in sensitivity between cell lines in more detail, the degrees of response of the cell lines to the same botulinum toxin treatment were compared by dividing the OD₄₅₀ₙₘ value of each group of the SiMa cell lines by the OD₄₅₀ₙₘ value of each group of SEPTIN2-3 cell lines. The results are shown in FIG. 11.

As shown in FIG. 11, it was confirmed that the OD450_{SEPTIN2}/OD450_{WT} value in the absence of toxin treatment is 1, whereas the value in the presence of toxin treatment increases to approximately 1.7, indicating a difference in sensitivity to the toxin between cell lines. Through this, it could be confirmed that the transduced cell line overexpressing the SEPTIN2 gene had increased sensitivity to botulinum toxin compared to the control (wild type).

### 6.3. Preparation of cell lysate of SiMa-thioredoxin cells treated with BoNT/A

In order to confirm a system for verifying the biological activity of a cell-based botulinum toxin, a cell lysate of cells treated with botulinum toxin was first prepared. To treat cells with botulinum toxin, a SiMa cell line as a control was dispensed into a 96-well plate containing a RPMI1640 medium supplemented with 10% FBS, 2 mM L-glutamine, and 1% penicillin-streptomycin at a concentration of 8 × 10⁴ cells/100 µL/well, and a SiMa-TXN3 cell line as an experimental group was dispensed into a 96-well plate containing a RPMI1640 medium supplemented with 10% FBS, 2 mM L-glutamine, 1% penicillin-streptomycin, and 1 µg/mL puromycin at a concentration of 8 × 10⁴ cells/100 µL/well. After each cell line was dispensed and cultured for 2 days, the media were removed and the cell line was treated with 100 µL of a differentiation medium supplemented with 1X B-27^{™} Plus Supplement (Gibco), 1X N-2 Supplement (Gibco), 2 mM L-glutamine and 25 µg/mL trisialoganglioside GT_{1B} (Matreya ) and cultured for 2 days to induce differentiation. After 2 days of differentiation, the medium was removed, and a BoNT/A complex was prepared in RPMI1640 at concentrations of 0, 1, 5, 10, 50, 100, 500, 1000 and 2000 (pM), and finally, 100 µL of each of the complexes was added to each well. After cells were treated with botulinum toxin and cultured for 4 days, the cells were lysed by removing the medium and treating each well with 110 µL of a lysis buffer (50 mM HEPES (pH 7.4), 150 mM NaCl, 1.5 mM MgClz and 1% Triton X-100) supplemented with a protease inhibitor cocktail. Then, the cell lysate was transferred to a 1.5 mL tube and centrifuged at 17,000 rpm and 4°C for 5 minutes to obtain the supernatant, thereby preparing a cell lysate for use in sandwich ELISA.

### 6.4. Confirmation of EC50 using sandwich ELISA

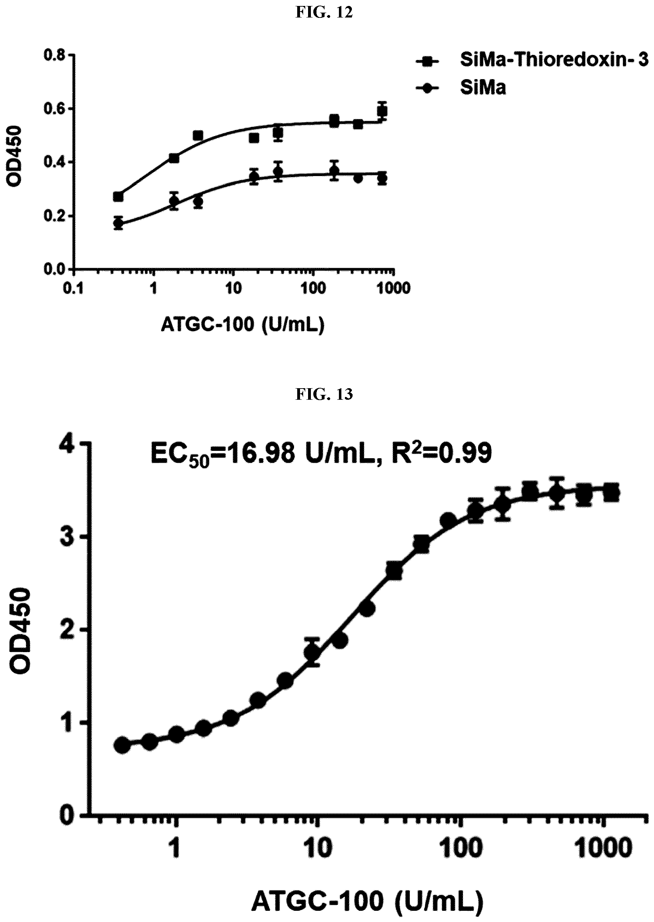

The sensitivity of the transduced cell line overexpressing the TXN gene to botulinum toxin was confirmed using the cell lysate of Example 6.3 in the same manner as in Example 6.2. The results are shown in FIG. 12.

As shown in FIG. 12, it could be confirmed that the transduced cell line overexpressing the thioredoxin gene had increased sensitivity to botulinum toxin compared to the control (wild type).

### Example 7: Verification of biological activity of BoNT/A pharmaceutical composition (Active Pharmaceutical Ingredient)

To confirm whether the system for verifying the biological activity of a cell-based botulinum toxin is also applicable to a botulinum toxin pharmaceutical composition (Active Pharmaceutical Ingredient), sandwich ELISA was performed using a SiMa-SEPTIN2-3 cell line. More specifically, the SiMa-SEPTIN2-3 cell line was dispensed into a 96-well plate containing a RPM1640 medium supplemented with 10% FBS, 2 mM L-glutamine, 1% penicillin-streptomycin, and 1 µg/mL puromycin at a concentration of 1.2 × 10⁵ cells/100 µL/well. Then, after the cell line was cultured for 2 days, the medium was removed and the cell line was treated with 100 µL of a differentiation medium supplemented with 1X B-27^{™} Plus Supplement, 1X N-2 Supplement, 2 mM L-glutamine and 25 µg/mL trisialoganglioside GT_{1B} and cultured for 2 days to induce differentiation. After 2 days of differentiation, the medium was removed, and a BoNT/A complex (botulinum toxin, which is a Active Pharmaceutical Ingredient in which other materials such as a preservative solution are removed from ATGC-100) was serially diluted 1.55-fold in RPMI1640 supplemented with 1X B-27^{™} Plus Supplement and 1X N-2 Supplement, and cells were treated with the complex at different concentrations. After cells were treated with botulinum toxin and cultured for 4 days, the cells were lysed by removing the medium and treating each well with 110 µL of a lysis buffer (50 mM HEPES (pH 7.4), 150 mM NaCl, 1.5 mM MgClz and 1% Triton X-100) supplemented with a protease inhibitor cocktail. Then, the cell lysate was transferred to a 1.5 mL tube and centrifuged at 17,000 rpm and 4°C for 5 minutes to obtain the supernatant, thereby preparing a cell lysate for use in sandwich ELISA.

Then, after a 25 kDa mouse synaptosomal protein (SNAP-25, a.a. 183-197) cleavage monoclonal antibody (Mybiosource), which is a capture antibody that specifically binds to cleaved SNAP-25, was diluted to a concentration of 0.8% (v/v) using a 0.1 M sodium carbonate coating buffer (pH 9.6), each well of a clear flat-bottom immuno nonsterile 96-well plate (Thermo Fisher) was treated with 100 µL of the antibody, and reacted without movement at 4°C for 16 hours. Thereafter, antibodies not bound to the wells were removed, and the wells were washed three times using 200 µL of 0.1% polysorbate 20 in PBS (PBST). Then, each well was treated with 200 µL of a blocking buffer (5% BSA PBST), and placed in a shaker under the conditions of room temperature and 75 rpm for 2 hour. After 2 hours, the blocking buffer was removed, 90 µL of the obtained cell lysate was added, and the mixture was reacted in a shaker under the conditions of room temperature and 75 rpm for 2 hours. Thereafter, the cell lysate was removed, and the cells were washed three times with 200 µL of PBST. Thereafter, after an anti-SNAP-25 antibody (Sigma-Aldrich, S9684), which is a detection antibody, was diluted to a concentration of 0.1% (v/v) using a blocking buffer, each well was treated with 100 µL of the diluted antibody, and reacted in a shaker under the conditions of room temperature and 75 rpm for 1 hour. Thereafter, all unbound antibodies were removed by washing three times using 200 µL of PBST, anti-rabbit HRP (abcam, ab6721), which is a secondary antibody, was diluted to 0.01% (v/v) using a blocking buffer, and each well was treated with 100 µL of the diluted antibody solution and reacted in a shaker under the conditions of room temperature and 75 rpm for 1 hour. Then, after the wells were washed three times using 200 µL of PBST, each well was treated with 100 µL of a TMB (ThermoFisher, 34028) solution, and then reacted at room temperature for 15 minutes. Thereafter, after each well was treated with 100 µL of 2N sulfuric acid, the absorbance (OD450) at 450 nm was measured using a SpectraMax Plus 384 Microplate Reader (Molecular Devices). A dose-response curve (four parameters) was drawn from acquired raw data using GraphPad Prism Version 7.00 (GraphPad Software, Inc.), and EC₅₀ values were calculated based on in-house mouse potency test results. The results are shown in FIG. 13.

As shown in FIG. 13, it was confirmed that the amount of SNAP-25 cleavage increased in a botulinum toxin dose-dependent manner. Also, it was confirmed that the EC₅₀ value was 16.98 U/mL.

### Example 8: Verification of biological activity of BoNT/A pharmaceutical composition (Finished Dosage Form)

To confirm whether the system for verifying the biological activity of a cell-based botulinum toxin is also applicable to another botulinum toxin pharmaceutical composition (Finished Dosage Form), sandwich ELISA was performed using a SiMa-SEPTIN2-3 cell line. More specifically, the SiMa-SEPTIN2-3 cell line was dispensed into a 96-well plate containing a RPM1640 medium supplemented with 10% FBS, 2 mM L-glutamine, 1% penicillin-streptomycin, and 1 µg/mL puromycin at a concentration of 1.2 × 10⁵ cells/100 µL/well. Then, after the cell line was cultured for 2 days, the medium was removed and the cell line was treated with 100 µL of a differentiation medium supplemented with 1X B-27^{™} Plus Supplement, 1X N-2 Supplement, 2 mM L-glutamine and 25 µg/mL trisialoganglioside GT_{1B} and cultured for 2 days to induce differentiation. After 2 days of differentiation, the medium was removed, and a BoNT/A Finished Dosage Form (ATGC-100) was serially diluted 1.4-fold in RPMI1640 supplemented with 1X B-27^{™} Plus Supplement and 1X N-2 Supplement, and cells were treated with the BoNT/A at different concentrations. After cells were treated with botulinum toxin and cultured for 4 days, the cells were lysed by removing the medium and treating each well with 110 µL of a lysis buffer (50 mM HEPES (pH 7.4), 150 mM NaCl, 1.5 mM MgClz and 1% Triton X-100) supplemented with a protease inhibitor cocktail. Then, the cell lysate was transferred to a 1.5 mL tube and centrifuged at 17,000 rpm and 4°C for 5 minutes to obtain the supernatant, thereby preparing a cell lysate for use in sandwich ELISA.

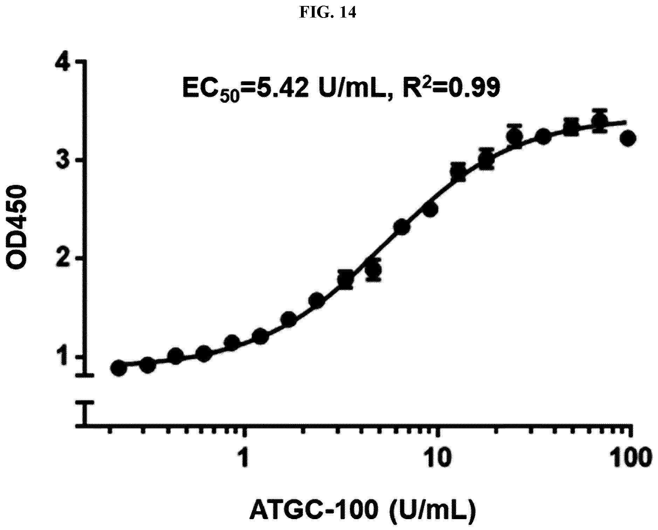

Then, after a 25 kDa mouse synaptosomal protein (SNAP-25, a.a. 183-197) cleavage monoclonal antibody (Mybiosource), which is a capture antibody that specifically binds to cleaved SNAP-25, was diluted to a concentration of 0.8% (v/v) using a 0.1 M sodium carbonate coating buffer (pH 9.6), each well of a clear flat-bottom immuno nonsterile 96-well plate (Thermo Fisher) was treated with 100 µL of the diluted antibody, and reacted without movement at 4°C for 16 hours. Thereafter, antibodies not bound to the wells were removed, and the wells were washed three times using 200 µL of 0.1% polysorbate 20 in PBS (PBST). Then, each well was treated with 200 µL of a blocking buffer (5% BSA PBST), and placed in a shaker under the conditions of room temperature and 75 rpm for 2 hour. After 2 hours, the blocking buffer was removed, 90 µL of the obtained cell lysate was added, and the mixture was reacted in a shaker under the conditions of room temperature and 75 rpm for 2 hours. Then, the cell lysate was removed, and the cells were washed three times with 200 µL of PBST. Thereafter, after an anti-SNAP-25 antibody (Sigma-Aldrich, S9684), which is a detection antibody, was diluted to a concentration of 0.1% (v/v) using a blocking buffer, each well was treated with 100 µL of the diluted antibody, and reacted in a shaker under the conditions of room temperature and 75 rpm for 1 hour. Thereafter, all unbound antibodies were removed by washing three times using 200 µL of PBST, anti-rabbit HRP (abcam, ab6721), which is a secondary antibody, was diluted to 0.01% (v/v) using a blocking buffer, and each well was treated with 100 µL of the diluted antibody solution and reacted in a shaker under the conditions of room temperature and 75 rpm for 1 hour. Then, after the wells were washed three times using 200 µL of PBST, each well was treated with 100 µL of a TMB (ThermoFisher, 34028) solution, and then reacted at room temperature for 15 minutes. Thereafter, after each well was treated with 100 µL of 2N sulfuric acid, the absorbance (OD450) at 450 nm was measured using a SpectraMax Plus 384 Microplate Reader (Molecular Devices). A dose-response curve (four parameters) was drawn from acquired raw data using GraphPad Prism Version 7.00 (GraphPad Software, Inc.), and EC₅₀ values were calculated based on in-house mouse potency test results. The results are shown in FIG. 14.

As shown in FIG. 14, it was confirmed that the amount of SNAP-25 cleavage increased in a botulinum toxin dose-dependent manner. Also, it was confirmed that the EC₅₀ value was 5.42 U/mL.

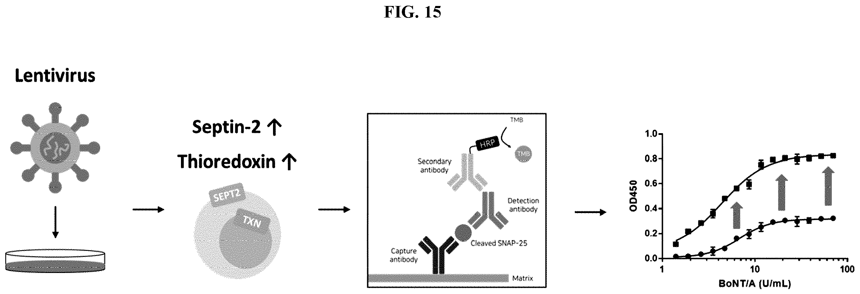

Through the above results, as shown in FIG. 15, it could be confirmed that by using a cell line overexpressing the SEPTIN2 or TXN gene using the lentivirus of the present invention, the potency of not only botulinum toxin, but also a botulinum toxin pharmaceutical composition, that is, a Active Pharmaceutical Ingredient and a Finished Dosage Form can be easily measured on the cell basis, and the sensitivity thereof can be remarkably improved. Therefore, it is expected that the cell line of the present invention can be applied to various industrial fields that use botulinum toxin.

The above-described description of the present invention is provided for illustrative purposes, and those skilled in the art to which the present invention pertains will understand that the present invention can be easily modified into other specific forms without changing the technical spirit or essential features of the present invention. Therefore, it should be understood that the above-described embodiments are only exemplary in all aspects and are not restrictive.

### [Industrial Applicability]

Since the cells for measuring botulinum toxin activity of the present invention have remarkably improved sensitivity to botulinum toxin, the cells can be widely used in various industrial fields to measure the activity of botulinum toxin as well as a botulinum toxin pharmaceutical composition in a Active Pharmaceutical Ingredient or Finished Dosage Form by replacing numerous animal experiments.

## Claims

1. Cells for measuring botulinum toxin activity, overexpressing SEPTIN2 or thioredoxin (TXN).

2. The cells of claim 1, wherein the cells are **characterized in that** a SEPTIN2 or TXN gene is transduced and overexpressed.

3. The cells of claim 1, wherein the cells have increased sensitivity to botulinum toxin intoxication.

4. The cells of claim 1, wherein the cells are any one selected from the group consisting of SiMa cells, LAN-2 cells, PC12 cells, Neuro-2a cells, LA1-55n cells, N18 cells, SH-SY5Y cells, Kelly cells, NB69 cells, N1E-115 cells, BE(2)-M17 cells, and SK-N-BE(2) cells.

5. The cells of claim 1, wherein the botulinum toxin is any one selected from the group consisting of botulinum toxin serotypes A, B, C, D, E, F, and G.

6. A method for measuring botulinum toxin activity, the method comprising the steps of: a) treating the cells of any one of claims 1 to 5 with botulinum toxin and culturing the cells;
b) lysing the cultured cells and collecting a cell lysate; and
c) measuring an amount of SNAP-25 cleavage product in the cell lysate.

7. The method of claim 6, wherein the amount of SNAP-25 cleavage product is measured using a sandwich immunoassay (ELISA) or Western blot.

8. A kit for measuring botulinum toxin activity, comprising the cells of any one of claims 1 to 5 as an active ingredient.
